(19) Europäisches Patentamt
European Patent Office
Office européen des brevets

(11) **EP 4 353 146 B1**

(12) # EUROPEAN PATENT SPECIFICATION

(45) Date of publication and mention
of the grant of the patent:
**27.08.2025 Bulletin 2025/35**

(21) Application number: **22819507.9**

(22) Date of filing: **07.06.2022**

(51) International Patent Classification (IPC):
**A61B 5/02** (2006.01)     **A61B 5/024** (2006.01)
**A61B 5/00** (2006.01)

(52) Cooperative Patent Classification (CPC):
**A61B 5/02416; A61B 5/721; A61B 5/725**

(86) International application number:
**PCT/CN2022/097309**

(87) International publication number:
**WO 2022/257909 (15.12.2022 Gazette 2022/50)**

(54) **SIGNAL PROCESSING METHOD AND APPARATUS, AND ELECTRONIC DEVICE**

SIGNALVERARBEITUNGSVERFAHREN UND -VORRICHTUNG SOWIE ELEKTRONISCHE VORRICHTUNG

PROCÉDÉ ET APPAREIL DE TRAITEMENT DE SIGNAL, ET DISPOSITIF ÉLECTRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.06.2021 CN 202110651546**

(43) Date of publication of application:
**17.04.2024 Bulletin 2024/16**

(73) Proprietor: **Vivo Mobile Communication Co., Ltd.**
**Dongguan, Guangdong 523863 (CN)**

(72) Inventor: **ZHONG, Lihao**
**Dongguan, Guangdong 523863 (CN)**

(74) Representative: **Lavoix**
**Bayerstraße 83**
**80335 München (DE)**

(56) References cited:
CN-A- 105 943 015     CN-A- 106 691 425
CN-A- 108 478 206     CN-A- 109 965 861
CN-A- 111 084 618     CN-A- 113 349 752

CN-A- 113 397 497     US-A1- 2009 005 695
US-A1- 2017 241 799   US-A1- 2019 008 458

• CHOWDHURY SAYEED SHAFAYET ET AL: "Real-Time Robust Heart Rate Estimation From Wrist-Type PPG Signals Using Multiple Reference Adaptive Noise Cancellation", IEEE JOURNAL OF BIOMEDICAL AND HEALTH INFORMATICS, vol. 22, no. 2, 1 March 2018 (2018-03-01), Piscataway, NJ, USA, pages 450 - 459, XP093204394, ISSN: 2168-2194, Retrieved from the Internet <URL:https://ieeexplore.ieee.org/stampPDF/getPDF.jsp?tp=&arnumber=7755741&ref=aHR0cHM6Ly9pZWVleHBsb3JlLmllZWWUub3JnL2RvY3VtZW50Lzc3NTU3NDE=> DOI: 10.1109/JBHI.2016.2632201

• FU GUANGMING, MU PING'AN; JIANG RUIJIE: "          Heart rate estimation from PPG signal based on LMS adaptive filter          ", ELECTRONIC MEASUREMENT TECHNOLOGY, vol. 42, no. 22, 1 January 2019 (2019-01-01), pages 160 - 164, XP093015555, ISSN: 1002-7300

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

This application belongs to the field of signal processing technologies, and in particular, to a signal processing method and apparatus, a readable storage medium and a computer program product.

### BACKGROUND

When a user is in motion, a pulse wave signal acquired by a device includes motion noise. If a heart rate of the user is estimated based on the pulse wave signal, a problem of relatively low estimation accuracy is caused.

Therefore, it is necessary to process the signal acquired by the device.

Document US2019/008458A1 discloses a method for extracting heart rate information.

Document US2009/005695A1 discloses a method for calculating a pulse rate.

Document CHOWDHURY SAYEED SHAFAYET ET AL: "Real-Time Robust Heart Rate Estimation From Wrist-Type PPG Signals Using Multiple Reference Adaptive Noise Cancellation" discloses a method for extracting heart rate information.

### SUMMARY

The invention is set out in the appended set of claims. An objective of embodiments of this application is to provide a signal processing method and apparatus, a readable storage medium and a computer program product, which can resolve a problem of relatively low estimation accuracy.

In the embodiments of this application, the first pulse wave signal and the motion signals in the multi-dimensional directions are obtained, where the first pulse wave signal includes motion noise; independent filtering processing is performed on the motion signal in each dimensional direction, and the noise estimated value corresponding to each dimensional direction is determined; and the noise estimated values in the multi-dimensional directions are used as the parallel input parameters, the first pulse wave signal is used as an input parameter, and filtering processing is performed on the first pulse wave signal, to obtain the second pulse wave signal. The embodiments of this application provide the signal processing method, which can resolve a problem of relatively low estimation accuracy.

### BRIEF DESCRIPTION OF THE DRAWINGS

FIG. 1 is a flowchart of a signal processing method according to an embodiment;
FIG. 2 is a block diagram of a signal flow according to an embodiment;
FIG. 3 is a block principle diagram of a signal processing apparatus according to an embodiment;
FIG. 4 is a schematic diagram of a hardware structure of an electronic device according to an embodiment; and
FIG. 5 is a schematic diagram of a hardware structure of another electronic device according to an embodiment.

### DETAILED DESCRIPTION

The technical solutions in the embodiments of this application are clearly described in the following with reference to the accompanying drawings in the embodiments of this application. Apparently, the described embodiments are some rather than all of the embodiments of this application. All other embodiments obtained by a person of ordinary skill in the art based on the embodiments of this application fall within the protection scope of this application.

The specification and claims of this application, and terms "first" and "second" are used to distinguish similar objects, but are not used to describe a specific sequence or order. It should be understood that the data termed in such a way are interchangeable in appropriate circumstances, so that the embodiments of this application can be implemented in orders other than the order illustrated or described herein. In addition, the objects distinguished by "first" and "second" are usually of a same type, without limiting a quantity of objects, for example, there may be one or more first objects. In addition, "and/or" in the description and the claims means at least one of the connected objects, and the character "/" in this specification generally indicates an "or" relationship between the associated objects.

A signal processing method provided in the embodiments of this application are described in detail below with reference to the accompanying drawings by using specific embodiments and application scenarios thereof.

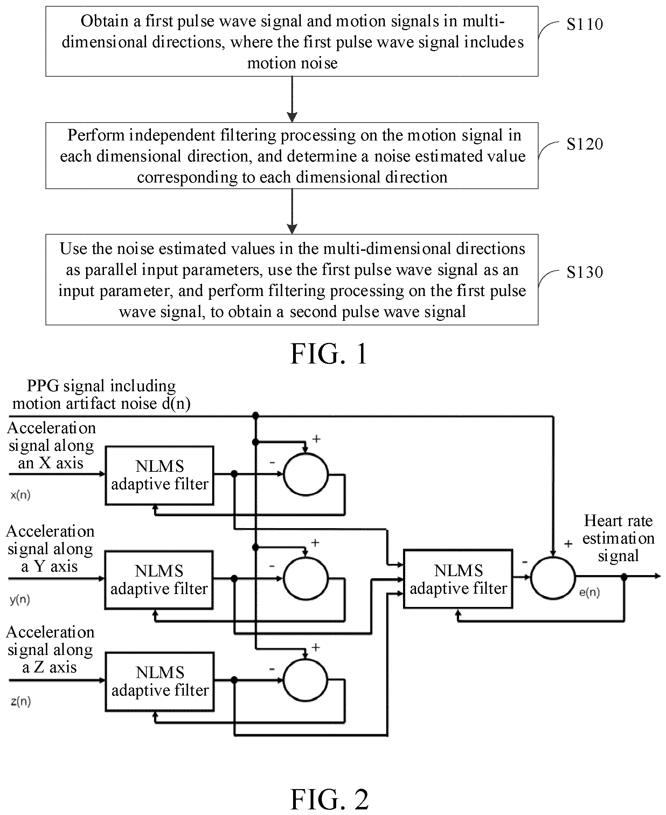

Referring to FIG. 1, a signal processing method provided in this embodiment may include the following step S110 to step S130.

Step S110: Obtain a first pulse wave signal and motion signals in multi-dimensional directions, where the first pulse wave signal includes motion noise.

In an embodiment of the present disclosure, the first pulse wave signal may be a pulse wave signal acquired by an electronic device, and the motion signals may be motion signals that are of the electronic device and that correspond to the multi-dimensional directions. The electronic device may be a smart wearable device such as a smartwatch or a smart band. A user may wear the electronic device during motion.

Specifically, a signal acquisition apparatus configured to acquire a pulse wave signal is arranged in the electronic device, and the acquired pulse wave signal includes motion noise or is referred to as noise including a motion artifact. In an embodiment of the present disclosure, the signal acquisition apparatus may be a photo plethysmo graphy (Photo Plethysmo Graphy, PPG) sensor and may acquire a PPG signal including motion noise. The PPG signal is, for example, $d(n)$ shown in FIG. 2.

$n$ may represent an $n^{th}$ processing cycle. The following is the same. Therefore, $d(n)$ is a pulse wave signal that corresponds to the $n^{th}$ processing cycle and that includes motion noise. The following is the same.

Specifically, a sensor configured to acquire motion signals that are of the electronic device and that correspond to the multi-dimensional directions may be arranged in the electronic device. In an embodiment of the present disclosure, the sensor may be a triaxial accelerometer and acquires motion signals of the electronic device that respectively correspond to an X axis direction, a Y axis direction, and a Z axis direction. The motion signals are, for example, $x(n)$, $y(n)$, and $z(n)$ that correspond to the $n^{th}$ processing cycle shown in FIG. 2.

Therefore, the multi-dimensional directions in this embodiment may be the X axis direction, the Y axis direction, and the Z axis direction.

After the first pulse wave signal including the motion noise and the motion signals in the multi-dimensional directions are obtained in step S110, the following step S120 may be performed.

Step S120: Perform independent filtering processing on the motion signal in each dimensional direction, and determine a noise estimated value corresponding to each dimensional direction.

In this step, independent filtering processing is performed on the motion signal in each dimensional direction, to implement independent and parallel processing on multi-channel motion signals. Based on this, the noise estimated value corresponding each dimensional direction may be determined.

In a feasible implementation, a plurality of filters may be designed. The plurality of filters are in a one-to-one correspondence with the multi-dimensional directions, and each filter independently performs filtering processing on a motion signal in a corresponding dimensional direction. Optionally, the filter may be an NLMS adaptive filter adopting a normalized least mean square (Normalized Least Mean Square, NLMS) algorithm shown in FIG. 2.

Specifically, each filter in the plurality of filters may perform filtering processing on a motion signal in a corresponding dimensional direction with reference to a filter coefficient thereof.

In this embodiment, the filter coefficient of each filter in the plurality of filters may be recorded as a filter coefficient corresponding to a corresponding dimensional direction.

Based on this, in an embodiment of the present disclosure, the performing independent filtering processing on the motion signal in each dimensional direction, and determining a noise estimated value corresponding to each dimensional direction in step S120 may include the following step S1201 and step S1202.

Step S1201: Determine, according to a motion signal in a first dimensional direction, a motion parameter corresponding to the first dimensional direction, where the first dimensional direction is any dimensional direction of the multi-dimensional directions.

In an embodiment of the present disclosure, the motion signal in the first dimensional direction includes: motion parameter values corresponding to the first dimensional direction at a series of sampling time points. At least some motion parameter values may be selected from the motion signal and are arranged according to a sequence of corresponding sampling time points, to obtain a motion parameter corresponding to the first dimensional direction. The motion parameter may be a vector.

In a feasible implementation, the some selected motion parameter values may be motion parameter values respectively corresponding to a plurality of sampling time points relatively closest to a current time. For example, when a motion parameter is constructed, the motion parameter is constructed by using motion parameter values acquired within first 1s to 2s.

In this embodiment, it is assumed that the motion signals that are of the electronic device and that correspond to the X axis direction, the Y axis direction, and the Z axis direction are respectively $x(n)$, $y(n)$, and $z(n)$, motion parameters $X(n)$, $Y(n)$, and $Z(n)$ respectively corresponding to the X axis direction, the Y axis direction, and the Z axis direction may be obtained.

Step S1202: Determine a noise estimated value corresponding to the first dimensional direction according to the motion parameter and a first filter coefficient corresponding to the first dimensional direction.

In the step, a noise estimated value in a corresponding dimensional direction is estimated with reference to the motion parameter and a filter coefficient of the filter. The noise estimated value may also be referred to as an adaptive estimated value of the first pulse wave signal d(n).

Referring to FIG. 2, when there are three-dimensional directions, three independent and parallel NLMS adaptive filters may be designed correspondingly, and each NLMS adaptive filter independently estimates a noise estimated value corresponding to a corresponding dimensional direction.

In a feasible implementation, the noise estimated value corresponding to each dimensional direction may be obtained through the following formula:

$$d_x(n)=w_x(n)*X(n);$$

$$d_y(n)=w_y(n)*Y(n);$$

and

$$d_z(n)=w_z(n)*Z(n),$$

where

n represents the $n^{th}$ processing cycle, $d_x$, $d_y$, and $d_z$ respectively represent noise estimated values corresponding to the X axis direction, the Y axis direction, and the Z axis direction of the triaxial accelerometer, $w_x$, $w_y$, and $w_z$ respectively represent filter coefficients corresponding to the X axis direction, the Y axis direction, and the Z axis direction of the triaxial accelerometer, X(n), Y(n), and Z(n) respectively represent motion parameters corresponding to the X axis direction, the Y axis direction, and the Z axis direction of the triaxial accelerometer, and a * operation represents multiplication.

After the noise estimated value corresponding to each dimensional direction is determined in step S120, the following step S130 may be performed.

Step S130: Use the noise estimated values in the multi-dimensional directions as parallel input parameters, use the first pulse wave signal as an input parameter, and perform filtering processing on the first pulse wave signal, to obtain a second pulse wave signal.

In a feasible implementation, another filter may be designed. The another filter has a fusion function and performs filtering processing on the first pulse wave signal with reference to the noise estimated value corresponding to each dimensional direction.

Referring to FIG. 2, the NLMS adaptive filter corresponding to each dimensional direction independently estimates a noise estimated value, the noise estimated value may be further inputted into an NLMS adaptive filter that has a fusion function, and the NLMS adaptive filter that has the fusion function may perform, with reference to each noise estimated value, filtering processing on the acquired first pulse wave signal that includes the motion noise, to obtain a second pulse wave signal, where the second pulse wave signal is a heart rate estimation signal e(n) shown in FIG. 2.

Specifically, the filter that has the fusion function may perform filtering processing on the first pulse wave signal with reference to a filter coefficient of the filter and the noise estimated value corresponding to each dimensional direction.

In this embodiment, in the plurality of filters that are in a one-to-one correspondence with the multi-dimensional directions, the filter coefficient of each filter may be recorded as a filter coefficient corresponding to a corresponding dimensional direction. For the fusion filter, a filter coefficient of the fusion filter may be recorded as a filter coefficient corresponding to the multi-dimensional directions.

Based on this, in an embodiment of the present disclosure, the using the noise estimated values in the multi-dimensional directions as parallel input parameters, using the first pulse wave signal as an input parameter, and performing filtering processing on the first pulse wave signal, to obtain a second pulse wave signal in step S130 may include the following step S1301 and step S1302.

Step S1301: Use the noise estimated values in the multi-dimensional direction as the parallel input parameters, use the first pulse wave signal as the input parameter, and obtain a second filter coefficient corresponding to the multi-dimensional directions, where the second filter coefficient includes a first filter coefficient value corresponding to each dimensional direction.

In a feasible implementation, the filter coefficient corresponding to the multi-dimensional directions may be obtained by using the following formula:

$$A(n)=[a_x(n), a_y(n), a_z(n)]',$$

where

n represents the $n^{th}$ processing cycle, A represents the filter coefficient corresponding to the multi-dimensional directions, and $a_x$, $a_y$, and $a_z$ respectively represent filter coefficient values corresponding to the X axis direction, the Y axis direction, and the Z axis direction of the triaxial accelerometer. $a_x(n)$, $a_y(n)$, and $a_z(n)$ are all scalars, and A(n) is a vector constructed by using the three scalars.

Step S1302: Perform filtering processing on the first pulse wave signal according to the noise estimated value in each dimensional direction and the first filter coefficient value corresponding to each dimensional direction, to obtain the second pulse wave signal.

Based on the foregoing content, in a feasible implementation, the second pulse wave signal may be obtained by using the following formula:

$$e(n)=d(n)-A(n)*P(n)=d(n)-[a_x(n)*d_x(n)+a_y(n)*d_y(n)+a_z(n)*d_z(n)];$$

$$A(n)=[a_x(n),\ a_y(n),\ a_z(n)]';$$

and

$$P(n)=[d_x(n),\ d_y(n),\ d_z(n)]',$$

where

n represents the $n^{th}$ processing cycle, e represents the second pulse wave signal, d represents the first pulse wave signal that includes the motion noise, A represents the filter coefficient corresponding to the multi-dimensional directions, P(n) represents a vector constructed by using three scalars $d_x(n)$, $d_y(n)$, and $d_z(n)$, $d_x$, $d_y$, and $d_z$ respectively represent noise estimated values corresponding to the X axis direction, the Y axis direction, and the Z axis direction of the triaxial accelerometer, $a_x$, $a_y$, and $a_z$ respectively represent filter coefficient values corresponding to the X axis direction, the Y axis direction, and the Z axis direction of the triaxial accelerometer, and a * operation represents multiplication.

In this embodiment, when filtering processing is performed on the first pulse wave signal that includes the motion noise based on the noise estimated value corresponding to each dimensional direction, a physiological signal may be separated from a noise signal generated during movement of a user, to obtain a second pulse wave signal in which noise has been removed. The signal may accurately reflect a change of a heart rate of the user, so that the heart rate of the user obtained through estimation is closer to an actual heart rate of the user, thereby improving heart rate estimation accuracy.

As shown in FIG. 2, the second pulse wave signal is e(n). The signal may be further used for heart rate estimation.

It can be learned from the foregoing content that in this embodiment, independent and parallel processing is performed on the motion signal corresponding to each dimensional direction, to estimate a noise estimated value in a corresponding dimensional direction, and then fusion filtering processing is performed based on the noise estimated value in each dimensional direction, to reduce noise, so as to obtain the second pulse wave signal. A heart rate of the user is estimated by using the second pulse wave signal, and an adverse effect of motion noise on the heart rate estimation can be eliminated, thereby improving heart rate estimation accuracy.

In addition, in this embodiment of the present disclosure, in the parallel manner, independent adaptive filtering can be performed on the first pulse wave signal that includes the motion noise by using a motion signal of each channel of a multi-channel accelerometer, to independently estimate the first pulse wave signal to obtain a corresponding noise estimated value, and then an adaptive approximate result of each channel can be combined in the fusion filter with a lowest loss function of the first pulse wave signal, to improve the denoising effect. Therefore, when the fusion filter has motion artifact noise, a convergence response speed of denoising can be accelerated based on the parallel denoising manner, and a signal-to-noise ratio of a pulse wave heart rate signal is improved.

In addition to the parallel filter manner provided in this embodiment of the present disclosure, filtering noise reduction processing may be further performed according to a cascading sequence. For an implementation in which filtering is performed according to the cascading sequence, for example, an X axis motion signal, a Y axis motion signal, and a Z axis motion signal of the triaxial accelerometer are used as input signals for noise reduction, and adaptive filters are combined according to a cascading sequence of X axis→Y axis-Z axis and sequentially perform adaptive filtering, to perform filtering processing on a pulse wave signal that includes motion noise, to achieve a noise reduction effect.

However, in the implementation in which filtering is performed according to the cascading sequence, adaptive filters correspond to channels are set in a cascading manner. Therefore, in the cascaded adaptive filters, filtering input signals have a sequence. Specifically, due to the sequence of the combined cascaded filters, a second-stage adaptive filter performs adaptive tracking according to a result outputted by a first-stage adaptive filter, and then a third-stage adaptive filter performs adaptive tracking according to a result outputted by the second-stage adaptive filter. Therefore, there is a sequence problem in an adaptive tracking sequence for calculation of a loss function, and an error signal of a latter-stage filter lags behind that of a previous-stage filter. Therefore, in a scenario in which a user wears a smartwatch and does a vigorous exercise such as running, the best effect cannot be necessarily achieved according to a specific cascading sequence (for example, X-Y-Z), and estimation and noise reduction on a heart rate signal by using an output signal are still inaccurate.

The parallel filtering solution provided in this embodiment of the present disclosure can resolve the problem. Specifically, the filters corresponding to the channels are set in a parallel manner. Therefore, in the parallel filters, filtering input signals have no sequence. Based on this, the filter corresponding to each axis of the accelerometer may simultaneously perform independent filtering processing on an input signal, to obtain a corresponding noise estimated value. An additional filter is arranged, and the filter fuses signals by using the noise estimated values as parallel input parameters, to perform filtering processing on the first pulse wave signal. During fusion, the fusion filter can further make a reasonable response according to a filtering status of each signal, to improve an input channel weight coefficient that can effectively reduce a loss function, thereby achieving an objective of multi-path parallel noise reduction.

In addition, in the implementation in which filtering is performed according to the cascading sequence, a reference signal of the second-stage adaptive filter uses an error signal $e(n)$ of the first-stage adaptive filter as an input. Therefore, in terms of an amplitude of a signal, the reference signal of the second-stage adaptive filter is smaller than that of the first-stage adaptive filter. Similarly, a reference signal of the third-stage adaptive filter is smaller than that of the second-stage adaptive filter. That is, there is a stage-by-stage attenuation problem in the combined cascaded filters.

However, the parallel filtering solution provided in this embodiment of the present disclosure does not have the problem. This is because independent parallel computing is performed on the input signals in this embodiment of the present disclosure, there is no problem in which a reference signal of a filter is an error signal outputted by a previous-stage filter of the filter in the combined cascaded filters. Therefore, the convergence speed can be improved. In addition, the filters perform parallel output, so that an amplitude of an estimation signal outputted by each channel is uniform, but information about an error signal of a lower-stage filter is always lower than that of a higher-stage filter in the cascaded filters.

In this embodiment of the present disclosure, a parallel filtering solution is provided, and the solution is correspondingly described by using the foregoing content. Based on the foregoing content, it should be noted that, filtering processing on the first pulse wave signal may be a periodical processing process. Therefore, to improve a noise reduction effect, a filter coefficient of each filter may be periodically updated. Therefore, when filtering processing is performed in each filtering processing cycle, a corresponding noise estimated value is estimated according to a current latest filter coefficient of each filter. Therefore, the first filter coefficient may be a real-time updated filter coefficient.

Based on this, to describe a possible implementation in which a filter coefficient corresponding to each dimensional direction is updated, in an embodiment of the present disclosure, the first filter coefficient is a filter coefficient that corresponds to a first processing cycle and that corresponds to the first dimensional direction.

For example, the first processing cycle is an $n^{th}$ processing cycle ($n$ is greater than or equal to 2). An X axis is used as an example, and the first filter coefficient corresponding to an X axis direction is $w_x(n)$.

Correspondingly, a filter coefficient that corresponds to the $n^{th}$ processing cycle and that corresponds to each dimensional direction may be obtained according to data corresponding to an $(n-1)^{th}$ processing cycle, to update the filter coefficient.

Based on this, before the determining a noise estimated value corresponding to the first dimensional direction according to the motion parameter and a first filter coefficient corresponding to the first dimensional direction in step S1202, the method further includes the following step A.

Step A: Obtain the first filter coefficient according to a target pulse wave signal that corresponds to a second processing cycle and that includes motion noise, a target motion parameter that corresponds to the second processing cycle and that corresponds to the first dimensional direction, and a third filter coefficient that corresponds to the second processing cycle and that corresponds to the first dimensional direction. The second processing cycle is a previous processing cycle adjacent to the first processing cycle.

Specifically, the first processing cycle is a current processing cycle, and the second processing cycle is a previous processing cycle of the current processing cycle. For example, when the first processing cycle is the $n^{th}$ processing cycle, the second processing cycle is the $(n-1)^{th}$ processing cycle.

For example, the first processing cycle is the $n^{th}$ processing cycle. The target pulse wave signal may be $d(n-1)$, the target motion parameter may be one of $X(n-1)$, $Y(n-1)$, and $Z(n-1)$, $X(n-1)$, $Y(n-1)$, and $Z(n-1)$ respectively correspond to the X axis direction, the Y axis direction, and the Z axis direction of the triaxial accelerometer, the third filter coefficient may be one of $w_x(n-1)$, $w_y(n-1)$, and $w_z(n-1)$, and $w_x(n-1)$, $w_y(n-1)$, and $w_z(n-1)$ respectively correspond to the X axis direction, the Y axis direction, and the Z axis direction of the triaxial accelerometer.

In this embodiment, for any dimensional direction, a filter coefficient that corresponds to a current processing cycle and that corresponds to the dimensional direction may be obtained according to a pulse wave signal that includes motion noise and that corresponds to a previous processing cycle, a motion parameter that corresponds to the previous processing cycle and that corresponds to the dimensional direction, and a filter coefficient that corresponds to the previous processing cycle and that corresponds to the dimensional direction. The process is repeated, and a filter coefficient corresponding to any dimensional direction can be updated in real time.

The filter coefficient corresponding to each dimensional direction is updated in real time with reference to a real-time changed pulse wave signal that includes motion noise and a real-time changed motion signal in each dimensional direction and based on an existing filter coefficient corresponding to each dimensional direction. Therefore, when a noise estimated value is calculated based on the real-time updated filter coefficient corresponding to each dimensional direction, accurate estimation can be performed on the noise estimated value, and accurate filtering processing is supported, thereby achieving a good noise filtering effect.

Based on the foregoing content, in an embodiment of the present disclosure, the obtaining the first filter coefficient according to a target pulse wave signal that corresponds to a second processing cycle and that includes motion noise, a target motion parameter that corresponds to the second processing cycle and that corresponds to the first dimensional direction, and a third filter coefficient that corresponds to the second processing cycle and that corresponds to the first dimensional direction in step A may include the following step A1 to step A3.

Step A1: Obtain an error signal corresponding to the first dimensional direction according to the target pulse wave signal, the target motion parameter, and the third filter coefficient.

Based on the foregoing content, for example, the X axis direction, the Y axis direction, or the Z axis direction of the triaxial accelerometer is the first dimensional direction. A noise estimated value that corresponds to the $(n-1)^{th}$ processing cycle and that corresponds to the X axis direction is $d_x(n-1)$, a noise estimated value that corresponds to the $(n-1)^{th}$ processing cycle and that corresponds to the Y axis direction is $d_y(n-1)$, and a noise estimated value that corresponds to the $(n-1)^{th}$ processing cycle and that corresponds to the Z axis direction is $d_z(n-1)$.

In the step, the X axis is used as an example, and an error signal $e_x(n-1)$ corresponding to the X axis direction may be obtained according to the target pulse wave signal $d(n-1)$, the target motion parameter $X(n-1)$, and the noise estimated value $d_x(n-1)$ that corresponds to the $(n-1)^{th}$ processing cycle and that corresponds to the X axis direction.

Similarly, an error signal $e_y(n-1)$ corresponding to the Y axis direction may be obtained, and an error signal $e_z(n-1)$ corresponding to the Z axis direction may be obtained.

In a feasible implementation, the error signal corresponding to each dimensional direction may be obtained through the following formula:

$$e_x(n-1)=d(n-1)-w_x(n-1)*X(n-1);$$

$$e_y(n-1)=d(n-1)-w_y(n-1)*Y(n-1);$$

and

$$e_z(n-1)=d(n-1)-w_z(n-1)*Z(n-1),$$

where
n-1 represents the $(n-1)^{d1}$ processing cycle, $e_x$, $e_y$, and $e_z$ respectively represent error signals corresponding to the X axis direction, the Y axis direction, and the Z axis direction of the triaxial accelerometer, d represents the first pulse wave signal that includes the motion noise, $w_x$, $w_y$, and $w_z$ respectively represent filter coefficients corresponding to the X axis direction, the Y axis direction, and the Z axis direction of the triaxial accelerometer, and X, Y, and Z respectively represent motion parameters corresponding to the X axis direction, the Y axis direction, and the Z axis direction of the triaxial accelerometer.

In another feasible implementation, the X axis is used as an example, because $d_x(n)=w_x(n)*X(n)$, the error signal corresponding to each dimensional direction may be obtained through the following formula.

$$e_x(n-1)=d(n-1)-d_x(n-1);$$

$$e_y(n-1)=d(n-1)-d_y(n-1);$$

and

$$e_z(n-1)=d(n-1)-d_z(n-1),$$

where
n-1 represents the $(n-1)^{th}$ processing cycle, $e_x$, $e_y$, and $e_z$ respectively represent error signals corresponding to the X axis direction, the Y axis direction, and the Z axis direction of the triaxial accelerometer, d represents the first pulse wave signal that includes the motion noise, and $d_x$, $d_y$, and $d_z$ respectively represent noise estimated values corresponding to the X axis direction, the Y axis direction, and the Z axis direction of the triaxial accelerometer.

It should be noted that, referring to step S120, it can be learned that to obtain the second pulse wave signal corresponding to the (n-1)th processing cycle, the noise estimated value that corresponds to the (n-1)th processing cycle and that corresponds to each dimensional direction needs to be estimated in advance. Therefore, when the error signal corresponding to the (n-1)th processing cycle is calculated, the error signal may be directly calculated according to the estimated noise estimated value rather than according to the filter coefficient and the motion parameter.

Step A2: Obtain a filter adjustment coefficient according to the error signal and the target motion parameter.

In the step, the filter adjustment coefficient is obtained according to the error signal and the motion parameter, and a current filter coefficient is adjusted based on the adjustment coefficient, to obtain a new filter coefficient.

In a feasible implementation, the filter adjustment coefficient corresponding to each dimensional direction may be obtained by using the following formula:

$$\Delta w_x(n-1)=2*\mu*e_x(n-1)*(X(n-1)/(X(n-1)*X(n-1))+\varepsilon);$$

$$\Delta w_y(n-1)=2*\mu*e_y(n-1)*(Y(n-1)/(Y(n-1)*Y(n-1))+\varepsilon);$$

and

$$\Delta w_z(n-1)=2*\mu*e_z(n-1)*(Z(n-1)/(Z(n-1)*Z(n-1))+\varepsilon),$$

where
n-1 represents the (n-1)th processing cycle, $\Delta w_x$, $\Delta w_y$, and $\Delta w_z$ respectively represent filter adjustment coefficients corresponding to the X axis direction, the Y axis direction, and the Z axis direction of the triaxial accelerometer, $e_x$, $e_y$, and $e_z$ respectively represent error signals corresponding to the X axis direction, the Y axis direction, and the Z axis direction of the triaxial accelerometer, X, Y, and Z respectively represent motion parameters corresponding to the X axis direction, the Y axis direction, and the Z axis direction of the triaxial accelerometer, and $\varepsilon$ is a constant.

Step A3: Obtain the first filter coefficient according to the filter adjustment coefficient and the third filter coefficient.

For example, the current processing cycle is the nth processing cycle, and the first filter coefficient is one of $w_x(n)$, $w_y(n)$, and $w_z(n)$. Correspondingly, the third filter coefficient is one of $w_x(n-1)$, $w_y(n-1)$, and $w_z(n-1)$.

Based on the foregoing content, in a feasible implementation, the filter coefficient corresponding to each dimensional direction may be obtained by using the following formula:

$$w_x(n)=w_x(n-1)+\Delta w_x(n-1);$$

$$w_y(n)=w_y(n-1)+\Delta w_y(n-1);$$

and

$$w_z(n)=w_z(n-1)+\Delta w_z(n-1),$$

where
n represents the nth processing cycle, n-1 represents the (n-1)d1 processing cycle, $w_x$, $w_y$, and $w_z$ respectively represent filter coefficients corresponding to the X axis direction, the Y axis direction, and the Z axis direction of the triaxial accelerometer, and $\Delta w_x$, $\Delta w_y$, and $\Delta w_z$ respectively represent filter adjustment coefficients corresponding to the X axis direction, the Y axis direction, and the Z axis direction of the triaxial accelerometer.

Therefore, in another embodiment of the present disclosure, after step A1, the filter coefficient corresponding to each dimensional direction may alternatively be directly calculated according to the following formula:

$$w_x(n)=w_x(n-1)+2*\mu*e_x(n-1)*(X(n-1)/(X(n-1)*X(n-1))+\varepsilon);$$

$$w_y(n)=w_y(n-1)+2*\mu*e_y(n-1)*(Y(n-1)/(Y(n-1)*Y(n-1))+\varepsilon);$$

and

$$w_z(n)=w_z(n-1)+2*\mu*e_z(n-1)*(Z(n-1)/(Z(n-1)*Z(n-1))+\varepsilon).$$

It can be learned from the foregoing content that in this embodiment of the present disclosure, the filter coefficient corresponding to each dimensional direction may be periodically updated, so that when filtering processing is periodically performed, the used filter coefficient corresponding to each dimensional direction is a real-time updated filter coefficient. Therefore, a noise reduction effect can be improved when filtering processing is performed based on the filter coefficient.

Based on a similar implementation principle, the filter coefficient of the fusion filter may also be periodically updated. Therefore, when filtering processing is performed on the first pulse wave signal that includes the motion noise, the real-time updated filter coefficient corresponding to the multi-dimensional directions may be used, so that the noise reduction effect is also improved when filtering processing is performed based on the filter coefficient.

Based on this, in an embodiment of the present disclosure, the second filter coefficient is a filter coefficient that corresponds to a first processing cycle and that corresponds to the multi-dimensional directions.

For example, the first processing cycle is the $n^{th}$ processing cycle (n is greater than or equal to 2). An X axis is used as an example, and the filter coefficient that corresponds to the $n^{th}$ processing cycle and that corresponds to the multi-dimensional directions is P(n).

Correspondingly, a filter coefficient of the fusion filter that corresponds to the $n^{th}$ processing cycle may be obtained according to data corresponding to the $(n-1)^{th}$ processing cycle, to update the filter coefficient corresponding to the multi-dimensional directions.

Based on this, the obtaining a second filter coefficient corresponding to the multi-dimensional directions in step S1301 includes: obtaining the second filter coefficient according to a pulse wave signal that corresponds to a second processing cycle and that is obtained through filtering processing, a fourth filter coefficient that corresponds to the second processing cycle and that corresponds to the multi-dimensional directions, and a noise estimated value that corresponds to the second processing cycle and that corresponds to each dimensional direction. The fourth filter coefficient includes a second filter coefficient value corresponding to each dimensional direction. The second processing cycle is a previous processing cycle adjacent to the first processing cycle.

Specifically, the first processing cycle is a current processing cycle, and the second processing cycle is a previous processing cycle of the current processing cycle. For example, when the first processing cycle is the $n^{th}$ processing cycle, the second processing cycle is the $(n-1)^{th}$ processing cycle.

In this embodiment, the filter coefficient corresponding to the multi-dimensional directions is updated according to a pulse wave signal e(n-1) that is obtained through filtering processing and that corresponds to the $(n-1)^{th}$ processing cycle, a filter coefficient A(n-1) that corresponds to the $(n-1)^{th}$ processing cycle and that corresponds to the multi-dimensional directions, and a noise estimated value $d_x(n-1)$, $d_y(n-1)$, and $d_z(n-1)$ that corresponds to the $(n-1)^{th}$ processing cycle and that corresponds to each dimensional direction, to obtain the filter coefficient that corresponds to the $n^{th}$ processing cycle and that corresponds to the multi-dimensional directions.

In this embodiment, the filter coefficient that corresponds to the current processing cycle and that corresponds to the multi-dimensional directions may be obtained according to the pulse wave signal that is obtained through filtering processing and that corresponds to the previous processing cycle, the filter coefficient that corresponds to the previous processing cycle and that corresponds to the multi-dimensional directions, and the noise estimated value that corresponds to the previous processing cycle and that corresponds to each dimensional direction. The process is repeated, and the filter coefficient corresponding to multi-dimensional directions can be updated in real time.

The filter coefficient is updated in real time with reference to the real-time changed pulse wave signal that is obtained through filtering processing and the real-time changed noise estimated values that corresponds to the dimensional directions and based on the existing filter coefficient that corresponds to the multi-dimensional directions. Therefore, when the second pulse wave signal is calculated based on the real-time updated filter coefficient that corresponds to the multi-dimensional directions, the second pulse wave signal can be accurately obtained, to achieve a better noise filtering effect.

In a feasible implementation, the filter coefficient corresponding to the multi-dimensional directions may be obtained by using the following formula:

$$A(n)=A(n-1)+2*\mu*e(n-1)*(P(n-1)/(P(n-1)*P(n-1))+\varepsilon);$$

$$A(n-1)=[a_x(n-1),\ a_y(n-1),\ a_z(n-1)]';$$

and

$$P(n-1)=[d_x(n-1),\ d_y(n-1),\ d_z(n-1)]',$$

.

where

n represents the $n^{th}$ processing cycle, n-1 represents the $(n-1)^{th}$ processing cycle, A represents the filter coefficient that corresponds to the multi-dimensional directions, $\mu$ is a step, e represents the second pulse wave signal, P(n-1) represents a vector constructed by using three scalars $d_x(n-1)$, $d_y(n-1)$, and $d_z(n-1)$, $d_x$, $d_y$, and $d_z$ respectively represent noise estimated values corresponding to the X axis direction, the Y axis direction, and the Z axis direction of the triaxial accelerometer, $a_y$, $a_y$, and $a_z$ respectively represent filter coefficient values corresponding to the X axis direction, the Y axis direction, and the Z axis direction of the triaxial accelerometer, a * operation represents multiplication, and $\varepsilon$ is a constant.

It can be learned from the foregoing content that in this embodiment of the present disclosure, the filter coefficient of the fusion filter may be periodically updated, so that when filtering processing is periodically performed, the used filter coefficient is a real-time updated filter coefficient that corresponds to the multi-dimensional directions. Therefore, a noise reduction effect can be improved when filtering processing is performed based on the filter coefficient.

It should be noted that, the signal processing method provided in the embodiments of this application may be performed by a signal processing apparatus or a control module configured to perform the signal processing method in the signal processing apparatus. In this embodiment of this application, the signal processing apparatus provided in this embodiment of this application is described by using an example in which the signal processing apparatus executes the signal processing method.

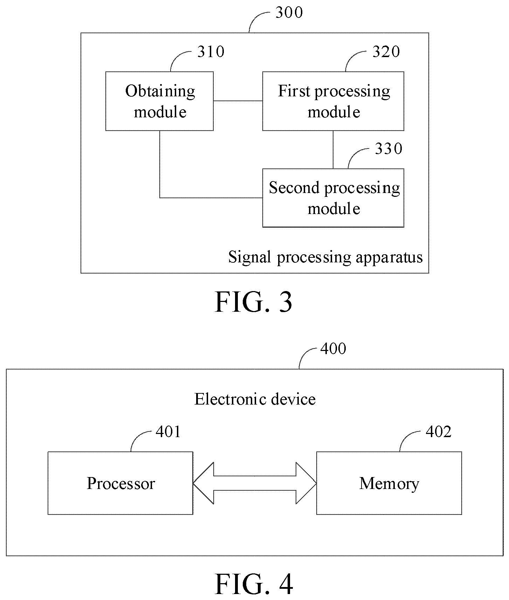

As shown in FIG. 3, a signal processing apparatus 300 provided in this embodiment may include: an obtaining module 310, a first processing module 320, and a second processing module 330.

The obtaining module 310 is configured to obtain a first pulse wave signal and motion signals in multi-dimensional directions, where the first pulse wave signal includes motion noise. The first processing module 320 is configured to perform independent filtering processing on the motion signal in each dimensional direction, and determine a noise estimated value corresponding to each dimensional direction. The second processing module 330 is configured to use the noise estimated values in the multi-dimensional directions as parallel input parameters, use the first pulse wave signal as an input parameter, and perform filtering processing on the first pulse wave signal, to obtain a second pulse wave signal.

In this embodiment, independent parallel processing is performed on the motion signal corresponding to each dimensional direction, to estimate a noise estimated value in a corresponding dimensional direction, and then fusion filtering processing is performed based on the noise estimated value in each dimensional direction, to reduce noise, so as to obtain the second pulse wave signal. A heart rate of the user is estimated by using the second pulse wave signal, and an adverse effect of motion noise on the heart rate estimation can be eliminated, thereby improving heart rate estimation accuracy.

In an embodiment of the present disclosure, the first processing module 320 is configured to determine, according to a motion signal in a first dimensional direction, a motion parameter corresponding to the first dimensional direction, where the first dimensional direction is any dimensional direction of the multi-dimensional directions; and determine a noise estimated value corresponding to the first dimensional direction according to the motion parameter and a first filter coefficient corresponding to the first dimensional direction.

In an embodiment of the present disclosure, the first filter coefficient is a filter coefficient that corresponds to a first processing cycle and that corresponds to the first dimensional direction. The first processing module 320 is configured to: before the determining a noise estimated value corresponding to the first dimensional direction according to the motion parameter and a first filter coefficient corresponding to the first dimensional direction, obtain the first filter coefficient according to a target pulse wave signal that corresponds to a second processing cycle and that includes motion noise, a target motion parameter that corresponds to the second processing cycle and that corresponds to the first dimensional direction, and a third filter coefficient that corresponds to the second processing cycle and that corresponds to the first dimensional direction. The second processing cycle is a previous processing cycle adjacent to the first processing cycle.

In an embodiment of the present disclosure, the first processing module 320 is configured to obtain an error signal corresponding to the first dimensional direction according to the target pulse wave signal, the target motion parameter, and the third filter coefficient; obtain a filter adjustment coefficient according to the error signal and the target motion parameter; and obtain the first filter coefficient according to the filter adjustment coefficient and the third filter coefficient.

In an embodiment of the present disclosure, the second processing module 330 is configured to use the noise estimated values in the multi-dimensional direction as the parallel input parameters, use the first pulse wave signal as the input parameter, and obtain a second filter coefficient corresponding to the multi-dimensional directions, where the second filter coefficient includes a first filter coefficient value corresponding to each dimensional direction; and perform filtering processing on the first pulse wave signal according to the noise estimated value in each dimensional direction and the first filter coefficient value corresponding to each dimensional direction, to obtain the second pulse wave signal.

In an embodiment of the present disclosure, the second filter coefficient is a filter coefficient that corresponds to a first processing cycle and that corresponds to the multi-dimensional directions. The second processing module 330 is configured to obtain the second filter coefficient according to a pulse wave signal that corresponds to a second processing cycle and that is obtained through filtering processing, a fourth filter coefficient that corresponds to the second processing cycle and that corresponds to the multi-dimensional directions, and a noise estimated value that corresponds to the second processing cycle and that corresponds to each dimensional direction. The fourth filter coefficient includes a second filter coefficient value corresponding to each dimensional direction. The second processing cycle is a previous processing cycle adjacent to the first processing cycle.

In an embodiment of the present disclosure, the first processing module 320 may include a plurality of first filters. The plurality of first filters are in a one-to-one correspondence with the multi-dimensional directions, and each first filter is configured to perform independent filtering processing on a motion signal in a corresponding dimensional direction and determine a noise estimated value corresponding to the corresponding dimensional direction.

In an embodiment of the present disclosure, the second processing module 330 may include a second filter. The second filter is configured to use the noise estimated values in the multi-dimensional directions as parallel input parameters, use the first pulse wave signal as an input parameter, and perform filtering processing on the first pulse wave signal, to obtain a second pulse wave signal.

The signal processing apparatus in this embodiment of this application may be an apparatus, or may be a component, an integrated circuit, or a chip in a terminal. The apparatus may be a mobile electronic device or may be a non-mobile electronic device. For example, the mobile electronic device may be a mobile phone, a tablet computer, a notebook computer, a palmtop computer, an in-vehicle electronic device, a wearable equipment, an ultra-mobile personal computer (ultra-mobile personal computer, UMPC), a netbook, a personal digital assistant (personal digital assistant, PDA), or the like. The non-mobile electronic device may be a server, a network attached storage (Network Attached Storage, NAS), a personal computer (personal computer, PC), a television (television, TV), a teller machine, a self-service machine, or the like. This is not specifically limited in this embodiment of this application.

The signal processing apparatus in this embodiment of this application may be an apparatus with an operating system. The operating system may be an Android (Android) operating system, an ios operating system, or another possible operating system. This is not specifically limited in this embodiment of this application.

The signal processing apparatus provided in this embodiment of this application can implement the processes implemented in the method embodiment of FIG. 1. To avoid repetition, details are not described herein again.

Optionally, as shown in FIG. 4, an embodiment of this application further provides an electronic device 400, including a processor 401, a memory 402, and a program or an instruction stored on the memory 402 and runnable on the processor 401. For example, the program or the instruction is executed by the processor 401 to implement each process of the foregoing embodiments of the signal processing method, and the same technical effects can be achieved. To avoid repetition, details are not described herein again.

The memory 402 may be configured to store a software program and various data. The memory 402 may mainly include a first storage area that includes the program or the instruction and a second storage area that includes data. The first storage area may store an operating system, an application program or instruction required by at least one function (for example, a sound playback function and an image display function), and the like. In addition, the memory 402 may include a volatile memory or a non-volatile memory, or the memory 402 may include both a volatile memory and a non-volatile memory. The non-volatile memory may be a read-only memory (Read-Only Memory, ROM), a programmable read-only memory (Programmable ROM, PROM), an erasable programmable read-only memory (Erasable PROM, EPROM), an electrically erasable programmable read-only memory (Electrically EPROM, EEPROM), or a flash memory. The volatile memory may be a random access memory (Random Access Memory, RAM), a static random access memory (Static RAM, SRAM), a dynamic random access memory (Dynamic RAM, DRAM), a synchronous dynamic random access memory (Synchronous DRAM, SDRAM), a double data rate synchronous dynamic random access memory (Double Data Rate SDRAM, DDRSDRAM), an enhanced synchronous dynamic random access memory (Enhanced SDRAM, ESDRAM), a synch link dynamic random access memory (Synch link DRAM, SLDRAM), and a direct rambus random access memory (Direct Rambus RAM, DRRAM). The memory 402 in this embodiment of this application includes but not limited to these memories and any other suitable types of memories.

The processor 401 may include one or more processing units. Optionally, the processor 401 may integrate an application processor and a modem. The application processor mainly processes an operation involved in an operating system, a user interface, an application program, and the like. The modem mainly processes a wireless communication signal, for example, a baseband processor. It may be understood that the foregoing modem may either not be integrated into the processor 401.

It should be noted that, the electronic device in this embodiment of this application includes the mobile electronic device and the non-mobile electronic device.

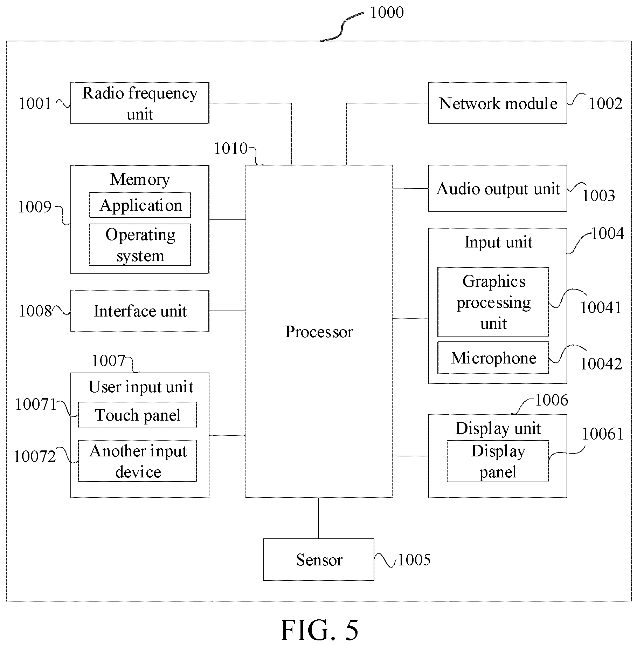

FIG. 5 is a schematic diagram of a hardware structure of an electronic device 1000 according to an embodiment of this application.

The electronic device 1000 includes, but is not limited to, components such as a radio frequency unit 1001, a network module 1002, an audio output unit 1003, an input unit 1004, a sensor 1005, a display unit 1006, a user input unit 1007, an interface unit 1008, a memory 1009, and a processor 1010.

A person skilled in the art may understand that the electronic device 1000 may further include the power supply (such as a battery) for supplying power to the components. The power supply may logically connect to the processor 1010 by using a power supply management system, thereby implementing functions, such as charging, discharging, and power consumption management, by using the power supply management system. The electronic device structure shown in FIG. 5 constitutes no limitation on an external device, and the electronic device may include more or fewer components than those shown in the figure, or some components may be combined, or a different component deployment may be used. Details are described herein again.

The processor 1010 is configured to obtain a first pulse wave signal and motion signals in multi-dimensional directions, where the first pulse wave signal includes motion noise; perform independent filtering processing on the motion signal in each dimensional direction, and determine a noise estimated value corresponding to each dimensional direction; and use the noise estimated values in the multi-dimensional directions as parallel input parameters, use the first pulse wave signal as an input parameter, and perform filtering processing on the first pulse wave signal, to obtain a second pulse wave signal.

In this embodiment, independent parallel processing is performed on the motion signal corresponding to each dimensional direction, to estimate a noise estimated value in a corresponding dimensional direction, and then fusion filtering processing is performed based on the noise estimated value in each dimensional direction, to reduce noise, so as to obtain the second pulse wave signal. A heart rate of the user is estimated by using the second pulse wave signal, and an adverse effect of motion noise on the heart rate estimation can be eliminated, thereby improving heart rate estimation accuracy.

Optionally, the processor 1010 is configured to determine, according to a motion signal in a first dimensional direction, a motion parameter corresponding to the first dimensional direction, where the first dimensional direction is any dimensional direction of the multi-dimensional directions; and determine a noise estimated value corresponding to the first dimensional direction according to the motion parameter and a first filter coefficient corresponding to the first dimensional direction.

Optionally, the first filter coefficient is a filter coefficient that corresponds to a first processing cycle and that corresponds to the first dimensional direction. The processor 1010 is configured to: before the determining a noise estimated value corresponding to the first dimensional direction according to the motion parameter and a first filter coefficient corresponding to the first dimensional direction, obtain the first filter coefficient according to a target pulse wave signal that corresponds to a second processing cycle and that includes motion noise, a target motion parameter that corresponds to the second processing cycle and that corresponds to the first dimensional direction, and a third filter coefficient that corresponds to the second processing cycle and that corresponds to the first dimensional direction. The second processing cycle is a previous processing cycle adjacent to the first processing cycle.

Optionally, the processor 1010 is configured to obtain an error signal corresponding to the first dimensional direction according to the target pulse wave signal, the target motion parameter, and the third filter coefficient; obtain a filter adjustment coefficient according to the error signal and the target motion parameter; and obtain the first filter coefficient according to the filter adjustment coefficient and the third filter coefficient.

Optionally, the processor 1010 is configured to use the noise estimated values in the multi-dimensional direction as the parallel input parameters, use the first pulse wave signal as the input parameter, and obtain a second filter coefficient corresponding to the multi-dimensional directions, where the second filter coefficient includes a first filter coefficient value corresponding to each dimensional direction; and perform filtering processing on the first pulse wave signal according to the noise estimated value in each dimensional direction and the first filter coefficient value corresponding to each dimensional direction, to obtain the second pulse wave signal.

Optionally, the second filter coefficient is a filter coefficient that corresponds to a first processing cycle and that corresponds to the multi-dimensional directions. The processor 1010 is configured to obtain the second filter coefficient according to a pulse wave signal that corresponds to a second processing cycle and that is obtained through filtering processing, a fourth filter coefficient that corresponds to the second processing cycle and that corresponds to the multi-dimensional directions, and a noise estimated value that corresponds to the second processing cycle and that corresponds to each dimensional direction. The fourth filter coefficient includes a second filter coefficient value corresponding to each dimensional direction. The second processing cycle is a previous processing cycle adjacent to the first processing cycle.

It should be understood that in this embodiment of this application, the input unit 1004 may include a graphics processing unit (Graphics Processing Unit, GPU) 10041 and a microphone 10042. The graphics processing unit 10041 performs processing on image data of a static picture or a video that is obtained by an image acquisition device (for example, a camera) in a video acquisition mode or an image acquisition mode. The display unit 1006 may include a display panel 10061, for example, the display panel 10061 configured in a form such as a liquid crystal display or an organic light-emitting diode. The user input unit 1007 includes a touch panel 10071 and another input device 10072. The touch panel 10071 is also referred to as a touchscreen. The touch panel 10071 may include two parts: a touch detection apparatus and a touch controller. The another input device 10072 may include, but not limited to, a physical keyboard, a functional key (such as a volume control key or a switch key), a track ball, a mouse, and a joystick, which are not described herein in detail. The memory 1009 may be configured to store a software program and various data, which includes, but not limited to, an application and an operating system. The processor 1010 may integrate an application processor and a modem processor. The application processor mainly processes an operating system, a user interface, an application program, and the like. The modem processor mainly processes wireless communication. It may be understood that the foregoing modem may either not be integrated into the processor 1010.

An embodiment of this application further provides a readable storage medium, storing a program or an instruction, where the program or the instruction is executed by a processor to implement each process of the foregoing embodiments of the signal processing method, and the same technical effect can be achieved. To avoid repetition, details are not repeated herein.

The processor is the processor in the electronic device in the foregoing embodiment. The readable storage medium includes a computer-readable storage medium such as a computer read-only memory (Read-Only Memory, ROM), a random access memory (Random Access Memory, RAM), a magnetic disk, or an optical disc.

An embodiment of this application further provides a chip, including a processor and a communication interface, where the communication interface is coupled to the processor, and the processor is configured to run a program or an instruction, to implement each process of the foregoing embodiments of the signal processing method, and the same technical effect can be achieved. To avoid repetition, details are not repeated herein.

It should be understood that, the chip mentioned in this embodiment of this application may also be referred to as a system-level chip, a system chip, a chip system, a system on chip, or the like.

It should be noted that, the terms "include", "including", or any other variation thereof in this specification is intended to cover a non-exclusive inclusion, which specifies the presence of stated processes, methods, objects, or apparatuses, but do not preclude the presence or addition of one or more other processes, methods, objects, or apparatuses. Without more limitations, elements defined by the sentence "including one" does not exclude that there are still other same elements in the processes, methods, objects, or apparatuses. In addition, it should be noted that, the scope of the methods and apparatuses in the implementations of this application is not limited to performing the functions in the order shown or discussed, but may further include performing the functions in a substantially simultaneous manner or in a reverse order depending on the functions involved. For example, the described methods may be performed in an order different from that described, and various steps may be added, omitted, or combined. In addition, features described with reference to some examples may be combined in other examples.

Through the descriptions of the foregoing implementations, a person skilled in the art may clearly understand that the methods in the foregoing embodiments may be implemented by means of software and a necessary general hardware platform, and certainly, may also be implemented by hardware, but in many cases, the former manner is a better implementation. Based on such an understanding, the technical solutions of this application essentially or the part contributing to the related art may be implemented in the form of a computer software product. The computer software product is stored in a storage medium (such as a ROM/RAM, a magnetic disk, or an optical disc), and includes several instructions for instructing a terminal (which may be a mobile phone, a computer, a server, a network device, or the like) to perform the method described in the embodiments of this application.

The embodiments of this application are described above with reference to the accompanying drawings. However, this application is not limited to the foregoing specific implementations. The foregoing specific implementations are illustrative instead of limitative.

**Claims**

1. A signal processing method, **characterized by** comprising:

obtaining (S110) a first pulse wave signal and motion signals in multi-dimensional directions, wherein the first pulse wave signal comprises motion noise, wherein the first pulse wave signal is the pulse wave information acquired by a signal acquisition apparatus of the electronic device, the motion signals in multi-dimensional directions are the motion signals in the corresponding multi-dimensional directions of the electronic device, acquired by a sensor of the electronic device;
performing (S120) independent filtering processing on the motion signal in each dimensional direction, and determining a noise estimated value corresponding to each dimensional direction; and
using (S130) the noise estimated values in the multi-dimensional directions as parallel input parameters, using the first pulse wave signal as an input parameter, and performing filtering processing on the first pulse wave signal, to obtain a second pulse wave signal;
wherein the using (S130) the noise estimated values in the multi-dimensional directions as parallel input parameters, using the first pulse wave signal as an input parameter, and performing filtering processing on the first pulse wave signal, to obtain a second pulse wave signal comprises:

using the noise estimated values in the multi-dimensional direction as the parallel input parameters, using the first pulse wave signal as the input parameter, and obtaining a (second) filter coefficient corresponding to the multi-dimensional directions, wherein the (second) filter coefficient comprises a first filter coefficient value corresponding to each dimensional direction; and

performing filtering processing on the first pulse wave signal according to the noise estimated value in each dimensional direction and the first filter coefficient value corresponding to each dimensional direction, to obtain the second pulse wave signal;

wherein the (second) filter coefficient is a filter coefficient that corresponds to a first processing cycle and that corresponds to the multi-dimensional directions; and

the obtaining the (second) filter coefficient corresponding to the multi-dimensional directions comprises:

obtaining the (second) filter coefficient according to a pulse wave signal that corresponds to a second processing cycle and that is obtained through filtering processing, a further (fourth) filter coefficient that corresponds to the second processing cycle and that corresponds to the multi-dimensional directions, and a noise estimated value that corresponds to the second processing cycle and that corresponds to each dimensional direction, wherein

the further (fourth) filter coefficient comprises a second filter coefficient value corresponding to each dimensional direction; and

the second processing cycle is a previous processing cycle adjacent to the first processing cycle.

2. The method according to claim 1, wherein the performing (S120) independent filtering processing on the motion signal in each dimensional direction, and determining a noise estimated value corresponding to each dimensional direction comprises:

determining, according to a motion signal in a first dimensional direction, a motion parameter corresponding to the first dimensional direction, wherein the first dimensional direction is any dimensional direction of the multi-dimensional directions; and

determining a noise estimated value corresponding to the first dimensional direction according to the motion parameter and a first filter coefficient corresponding to the first dimensional direction.

3. The method according to claim 2, wherein the first filter coefficient is a filter coefficient that corresponds to the first processing cycle and that corresponds to the first dimensional direction; and

before the determining a noise estimated value corresponding to the first dimensional direction according to the motion parameter and a first filter coefficient corresponding to the first dimensional direction, the method further comprises:

obtaining the first filter coefficient according to a target pulse wave signal that corresponds to the second processing cycle and that comprises motion noise, a target motion parameter that corresponds to the second processing cycle and that corresponds to the first dimensional direction, and a third filter coefficient that corresponds to the second processing cycle and that corresponds to the first dimensional direction.

4. The method according to claim 3, wherein the obtaining the first filter coefficient according to a target pulse wave signal that corresponds to a second processing cycle and that comprises motion noise, a target motion parameter that corresponds to the second processing cycle and that corresponds to the first dimensional direction, and a third filter coefficient that corresponds to the second processing cycle and that corresponds to the first dimensional direction comprises:

obtaining an error signal corresponding to the first dimensional direction according to the target pulse wave signal, the target motion parameter, and the third filter coefficient;

obtaining a filter adjustment coefficient according to the error signal and the target motion parameter; and

obtaining the first filter coefficient according to the filter adjustment coefficient and the third filter coefficient.

5. A signal processing apparatus (300), **characterized by** comprising:

an obtaining module (310), configured to obtain a first pulse wave signal and motion signals in multi-dimensional directions, wherein the first pulse wave signal comprises motion noise,

wherein the first pulse wave signal is the pulse wave information acquired by a signal acquisition apparatus of the electronic device, the motion signals in multi-dimensional directions are the motion signals in the corresponding multi-dimensional directions of the electronic device, acquired by a sensor of the electronic device;

a first processing module (320), configured to perform independent filtering processing on the motion signal in each dimensional direction, and determine a noise estimated value corresponding to each dimensional direction; and

a second processing module (330), configured to use the noise estimated values in the multi-dimensional directions as parallel input parameters, use the first pulse wave signal as an input parameter, and perform filtering processing on the first pulse wave signal, to obtain a second pulse wave signal;

wherein the second processing module (330) is configured to use the noise estimated values in the multi-dimensional direction as the parallel input parameters, use the first pulse wave signal as the input parameter, and obtain a (second) filter coefficient corresponding to the multi-dimensional directions, wherein the (second) filter coefficient comprises a first filter coefficient value corresponding to each dimensional direction; and perform filtering processing on the first pulse wave signal according to the noise estimated value in each dimensional direction and the first filter coefficient value corresponding to each dimensional direction, to obtain the second pulse wave signal;

wherein the (second) filter coefficient is a filter coefficient that corresponds to a first processing cycle and that corresponds to the multi-dimensional directions; and

the second processing module (330) is configured to obtain the (second) filter coefficient according to a pulse wave signal that corresponds to a second processing cycle and that is obtained through filtering processing, a further (fourth) filter coefficient that corresponds to the second processing cycle and that corresponds to the multi-dimensional directions, and a noise estimated value that corresponds to the second processing cycle and that corresponds to each dimensional direction, wherein the further (fourth) filter coefficient comprises a second filter coefficient value corresponding to each dimensional direction; and the second processing cycle is a previous processing cycle adjacent to the first processing cycle.

6. The apparatus (300) according to claim 5, wherein the first processing module (320) is configured to determine, according to a motion signal in a first dimensional direction, a motion parameter corresponding to the first dimensional direction, wherein the first dimensional direction is any dimensional direction of the multi-dimensional directions; and determine a noise estimated value corresponding to the first dimensional direction according to the motion parameter and a first filter coefficient corresponding to the first dimensional direction.

7. The apparatus (300) according to claim 6, wherein the first filter coefficient is a filter coefficient that corresponds to the first processing cycle and that corresponds to the first dimensional direction; and the first processing module (320) is configured to: before the determining a noise estimated value corresponding to the first dimensional direction according to the motion parameter and a first filter coefficient corresponding to the first dimensional direction, obtain the first filter coefficient according to a target pulse wave signal that corresponds to the second processing cycle and that comprises motion noise, a target motion parameter that corresponds to the second processing cycle and that corresponds to the first dimensional direction, and a third filter coefficient that corresponds to the second processing cycle and that corresponds to the first dimensional direction.

8. The apparatus (300) according to claim 7, wherein the first processing module (320) is configured to obtain an error signal corresponding to the first dimensional direction according to the target pulse wave signal, the target motion parameter, and the third filter coefficient; obtain a filter adjustment coefficient according to the error signal and the target motion parameter; and obtain the first filter coefficient according to the filter adjustment coefficient and the third filter coefficient.

9. A readable storage medium storing a program or instruction which, when executed by a computer, cause the computer to carry out the steps of the signal processing method according to any one of claims 1 to 4.

10. A computer program product comprising instructions which, when the program is executed by a computer, cause the computer to carry out the signal processing method according to any one of claims 1 to 4.

**Patentansprüche**

1. Signalverarbeitungsverfahren, **dadurch gekennzeichnet, dass** es Folgendes umfasst:

Erlangen (S110) eines ersten Impulswellensignals und von Bewegungssignalen in mehrdimensionalen Richtungen, wobei das erste Impulswellensignal Bewegungsrauschen umfasst,

wobei das erste Impulswellensignal

die Impulswelleninformationen sind, die von einem Signalerfassungsgerät der elektronischen Vorrichtung erfasst werden,

die Bewegungssignale in mehrdimensionalen Richtungen die Bewegungssignale in den entsprechenden mehrdimensionalen Richtungen der elektronischen Vorrichtung sind, die von einem Sensor der elektronischen Vorrichtung erfasst werden;

Durchführen (S120) einer unabhängigen Filterverarbeitung an dem Bewegungssignal in jeder dimensionalen Richtung und Bestimmen eines geschätzten Rauschwerts, der jeder dimensionalen Richtung entspricht; und

Verwenden (S130) der geschätzten Rauschwerte in den mehrdimensionalen Richtungen als parallele Eingangsparameter, Verwenden des ersten Impulswellensignals als Eingangsparameter und Durchführen eines Filterns des ersten Impulswellensignals, um ein zweites Impulswellensignal zu erlangen;

wobei das Verwenden (S130) der geschätzten Rauschwerte in den mehrdimensionalen Richtungen als parallele Eingangsparameter, Verwenden des ersten Impulswellensignals als Eingangsparameter und Durchführen eines Filterns des ersten Impulswellensignals, um ein zweites Impulswellensignal zu erlangen, Folgendes umfasst:

Verwenden der geschätzten Rauschwerte in der mehrdimensionalen Richtung als parallele Eingangsparameter, Verwenden des ersten Impulswellensignals als Eingangsparameter und Erlangen eines (zweiten)

Filterkoeffizienten, der den mehrdimensionalen Richtungen entspricht, wobei der (zweite) Filterkoeffizient

einen ersten Filterkoeffizientwert umfasst, der jeder dimensionalen Richtung entspricht; und

Durchführen einer Filterverarbeitung des ersten Impulswellensignals, das dem geschätzten Rauschwert entspricht, in jeder dimensionalen Richtung und des ersten Filterkoeffizientwerts, der jeder dimensionalen Richtung entspricht, um das zweite Impulswellensignal zu erlangen;

wobei der (zweite)

Filterkoeffizient ein Filterkoeffizient ist, der einem ersten Verarbeitungszyklus entspricht und der den mehrdimensionalen Richtungen entspricht; und

das Erlangen des (zweiten)

Filterkoeffizienten, der den mehrdimensionalen Richtungen entspricht, Folgendes umfasst:

Erlangen des (zweiten)

Filterkoeffizienten gemäß einem Impulswellensignal, das

einem zweiten Verarbeitungszyklus entspricht und das durch Filterverarbeitung erlangt wird, eines weiteren (vierten)

Filterkoeffizienten, der dem zweiten Verarbeitungszyklus entspricht und der den mehrdimensionalen Richtungen entspricht, und eines geschätzten Rauschwerts, der dem zweiten Verarbeitungszyklus entspricht und der jeder dimensionalen Richtung entspricht, wobei der weitere (vierte) Filterkoeffizient einen zweiten

Filterkoeffizientwert umfasst, der jeder dimensionalen Richtung entspricht; und

der zweite Verarbeitungszyklus ein vorheriger Verarbeitungszyklus ist, der an den ersten Verarbeitungszyklus angrenzt.

2. Verfahren nach Anspruch 1, wobei das Durchführen (S120) einer unabhängigen Filterverarbeitung an dem Bewegungssignal in jeder dimensionalen Richtung und das Bestimmen eines geschätzten Rauschwerts, der jeder dimensionalen Richtung entspricht, Folgendes umfasst:

Bestimmen, gemäß einem Bewegungssignal in einer ersten dimensionalen Richtung ,eines Bewegungsparameters, der der ersten dimensionalen Richtung entspricht, wobei die erste dimensionale Richtung eine beliebige dimensionale Richtung der mehrdimensionalen Richtungen ist; und

Bestimmen eines geschätzten Rauschwerts, der der ersten dimensionalen Richtung entspricht, gemäß dem Bewegungsparameter und einem ersten Filterkoeffizienten, der der ersten dimensionalen Richtung entspricht.

3. Verfahren nach Anspruch 2, wobei der erste Filterkoeffizient ein Filterkoeffizient ist, der dem ersten Verarbeitungszyklus entspricht und der der ersten dimensionalen Richtung entspricht; und

wobei das Verfahren vor dem Bestimmen eines geschätzten Rauschwerts, der der ersten dimensionalen Richtung entspricht, gemäß dem Bewegungsparameter und einem ersten Filterkoeffizienten, der der ersten dimensionalen Richtung entspricht, ferner Folgendes umfasst:

Erlangen des ersten Filterkoeffizienten gemäß einem Ziel-Impulswellensignal, das dem zweiten Verarbeitungszyklus entspricht und das Bewegungsrauschen umfasst, einem Ziel-Bewegungsparameter, der dem zweiten Verarbeitungszyklus entspricht und der der ersten dimensionalen Richtung entspricht, und einem dritten Filterkoeffizienten, der dem zweiten Verarbeitungszyklus entspricht und der der ersten dimensionalen Richtung entspricht.

**4.** Verfahren nach Anspruch 3, wobei das Erlangen des ersten Filterkoeffizienten gemäß einem Ziel-Impulswellensignal, das einem zweiten Verarbeitungszyklus entspricht und das Bewegungsrauschen umfasst, einem Ziel-Bewegungsparameter, der dem zweiten Verarbeitungszyklus entspricht und der der ersten dimensionalen Richtung entspricht, und einem dritten Filterkoeffizienten, der dem zweiten Verarbeitungszyklus entspricht und der der ersten dimensionalen Richtung entspricht, Folgendes umfasst:

Erlangen eines Fehlersignals, das der ersten dimensionalen Richtung entspricht, gemäß dem Ziel-Impulswellensignal, dem Ziel-Bewegungsparameter und dem dritten Filterkoeffizienten;
Erlangen eines Filteranpassungskoeffizienten gemäß dem Fehlersignal und dem Ziel-Bewegungsparameter; und
Erlangen des ersten Filterkoeffizienten gemäß dem Filteranpassungskoeffizienten und dem dritten Filterkoeffizienten.

**5.** Signalverarbeitungsgerät (300), **dadurch gekennzeichnet, dass** es Folgendes umfasst:

ein Erlangungsmodul (310), das konfiguriert ist, um ein erstes Impulswellensignal und Bewegungssignale in mehrdimensionalen Richtungen zu erlangen, wobei das erste Impulswellensignal Bewegungsrauschen umfasst,
wobei das erste Impulswellensignal die Impulswelleninformationen sind, die von einem Signalerfassungsgerät der elektronischen Vorrichtung erfasst werden,
die Bewegungssignale in mehrdimensionalen
Richtungen die Bewegungssignale in den entsprechenden mehrdimensionalen Richtungen der elektronischen Vorrichtung sind, die von einem Sensor der elektronischen Vorrichtung erfasst werden;
ein erstes Verarbeitungsmodul
(320), das konfiguriert ist, um eine unabhängige Filterverarbeitung des Bewegungssignals in jeder dimensionalen Richtung durchzuführen und einen geschätzten Rauschwert zu bestimmen, der jeder dimensionalen Richtung entspricht; und
ein zweites Verarbeitungsmodul (330), das konfiguriert ist, um die geschätzten Rauschwerte in den mehrdimensionalen Richtungen als parallele Eingangsparameter zu verwenden, das erste Impulswellensignal als einen Eingangsparameter zu verwenden und eine Filterverarbeitung des ersten Impulswellensignals durchzuführen, um ein zweites Impulswellensignal zu erlangen;
wobei das zweite Verarbeitungsmodul (330) konfiguriert ist, um die geschätzten Rauschwerte in der mehrdimensionalen Richtung als die parallelen Eingangsparameter zu verwenden, das erste Impulswellensignal als Eingangsparameter zu verwenden und einen (zweiten)
Filterkoeffizienten zu erlangen, der den
mehrdimensionalen Richtungen entspricht, wobei der (zweite)
Filterkoeffizient einen ersten
Filterkoeffizientwert umfasst, der jeder dimensionalen Richtung entspricht; und Durchführen einer Filterverarbeitung des ersten Impulswellensignals, das dem geschätzten Rauschwert entspricht, in jeder dimensionalen Richtung und des ersten Filterkoeffizientwerts, der jeder dimensionalen Richtung entspricht, um das zweite Impulswellensignal zu erlangen;
wobei der (zweite)
Filterkoeffizient ein Filterkoeffizient ist, der einem ersten
Verarbeitungszyklus entspricht und der den mehrdimensionalen Richtungen entspricht; und
das zweite Verarbeitungsmodul (330) konfiguriert ist, um den (zweiten)
Filterkoeffizienten
gemäß einem Impulswellensignal zu erlangen, das einem zweiten Verarbeitungszyklus entspricht und das durch Filterverarbeitung erlangt wird, eines weiteren (vierten)
Filterkoeffizienten, der dem zweiten
Verarbeitungszyklus entspricht und der den mehrdimensionalen Richtungen entspricht, und eines Rauschschätzwerts, der dem zweiten Verarbeitungszyklus entspricht und der jeder dimensionalen Richtung entspricht, wobei der weitere (vierte) Filterkoeffizient einen zweiten Filterkoeffizientwert
umfasst, der jeder dimensionalen Richtung entspricht; und der zweite Verarbeitungszyklus ein vorheriger Verarbeitungszyklus ist, der an den ersten Verarbeitungszyklus angrenzt.

6. Gerät (300) nach Anspruch 5, wobei das erste Verarbeitungsmodul (320) konfiguriert ist, um gemäß einem Bewegungssignal in einer ersten dimensionalen Richtung einen Bewegungsparameter zu bestimmen, der der ersten dimensionalen Richtung entspricht, wobei der ersten dimensionalen Richtung eine beliebige dimensionale Richtung der mehrdimensionalen Richtungen ist; und einen geschätzten Rauschwert, der der ersten dimensionalen Richtung entspricht, gemäß dem Bewegungsparameter und einem ersten Filterkoeffizienten, der der ersten dimensionalen Richtung entspricht, zu bestimmen.

7. Gerät (300) nach Anspruch 6, wobei der erste Filterkoeffizient ein Filterkoeffizient ist, der dem ersten Verarbeitungszyklus entspricht und der der ersten dimensionalen Richtung entspricht; und
das erste Verarbeitungsmodul (320) zu Folgendem konfiguriert ist: vor dem Bestimmen eines geschätzten Rauschwerts, der der ersten dimensionalen Richtung entspricht, gemäß dem Bewegungsparameter und einem ersten Filterkoeffizienten, der der ersten dimensionalen Richtung entspricht, Erlangen des ersten Filterkoeffizienten gemäß einem Zielimpulswellensignal, das dem zweiten Verarbeitungszyklus entspricht und das Bewegungsrauschen umfasst, eines Ziel-Bewegungsparameters, der dem zweiten Verarbeitungszyklus entspricht und der der ersten dimensionalen Richtung entspricht, und eines dritten Filterkoeffizienten, der dem zweiten Verarbeitungszyklus entspricht und der der ersten dimensionalen Richtung entspricht.

8. Vorrichtung (300) nach Anspruch 7, wobei das erste Verarbeitungsmodul (320) konfiguriert ist, um ein Fehlersignal, das der ersten dimensionalen Richtung entspricht, gemäß dem Ziel-Impulswellensignal, dem Ziel-Bewegungsparameter und dem dritten Filterkoeffizienten zu erlangen; einen Filteranpassungskoeffizienten gemäß dem Fehlersignal und dem Ziel-Bewegungsparameter zu erlangen; und den ersten Filterkoeffizienten gemäß dem Filteranpassungskoeffizienten und dem dritten Filterkoeffizienten zu erlangen.

9. Lesbares Speichermedium, das ein Programm oder eine Anweisung speichert, die, wenn sie von einem Computer ausgeführt werden, den Computer veranlassen, die Schritte des Signalverarbeitungsverfahrens nach einem der Ansprüche 1 bis 4 auszuführen.

10. Computerprogrammprodukt mit Befehlen, die, wenn

das Programm von einem Computer ausgeführt wird, den Computer veranlassen,
das Signalverarbeitungsverfahren nach einem der Ansprüche 1 bis 4 durchzuführen.

## Revendications

1. Procédé de traitement des signaux, **caractérisé en ce qu'**il comprend :

l'obtention (S110) d'un premier signal d'onde d'impulsion et des signaux de mouvement dans des directions multidimensionnelles, le premier signal d'onde d'impulsion comprenant un bruit de mouvement,
dans lequel le premier signal
d'onde d'impulsion est l'information d'onde d'impulsion acquise par un appareil d'acquisition de signaux du dispositif électronique,
les signaux de mouvement dans les directions multidimensionnelles sont les signaux de mouvement dans les directions multidimensionnelles correspondantes du dispositif électronique, acquis par un capteur du dispositif électronique ;
la réalisation (S120) d'un traitement de filtrage indépendant sur le signal de mouvement dans chaque direction dimensionnelle, et la détermination d'une valeur estimée de bruit correspondant à chaque direction dimensionnelle ; et
l'utilisation (S130) des valeurs estimées du bruit dans les directions multidimensionnelles comme paramètres d'entrée parallèles, l'utilisation du premier signal d'onde d'impulsion comme paramètre d'entrée et la réalisation d'un traitement de filtrage sur le premier signal d'onde d'impulsion, pour obtenir un deuxième signal d'onde d'impulsion ;
dans lequel l'utilisation (S130) des valeurs estimées du bruit dans les directions multidimensionnelles comme paramètres d'entrée parallèles, l'utilisation du premier signal d'onde d'impulsion comme paramètre d'entrée et la réalisation d'un traitement de filtrage sur le premier signal d'onde d'impulsion, pour obtenir un deuxième signal d'onde d'impulsion, comprennent :

l'utilisation des valeurs estimées du bruit dans la direction multidimensionnelle comme paramètres d'entrée parallèles, l'utilisation du premier signal d'onde d'impulsion comme paramètre d'entrée, et l'obtention d'un (deuxième)

coefficient de filtrage correspondant aux directions multidimensionnelles, dans lequel le (deuxième) coefficient de

filtrage comprend une première valeur de coefficient de filtrage correspondant à chaque direction dimensionnelle ; et

la réalisation d'un traitement de filtrage sur le premier signal d'onde d'impulsion en fonction de la valeur estimée du bruit dans chaque direction dimensionnelle et de la valeur du premier coefficient de filtrage correspondant à chaque direction dimensionnelle, afin d'obtenir le deuxième signal d'onde d'impulsion ; dans lequel le (deuxième)

coefficient de filtrage est un coefficient de filtrage qui correspond à un premier cycle de traitement et qui correspond aux directions multidimensionnelles ; et

l'obtention du (deuxième)

coefficient de filtrage correspondant aux directions multidimensionnelles comprend :

l'obtention du (deuxième)

coefficient de filtrage en fonction d'un signal d'onde d'impulsion correspondant à un deuxième cycle de traitement et qui est obtenu par un traitement de filtrage, un autre (quatrième) coefficient de filtrage qui correspond au deuxième cycle de traitement et qui correspond aux directions multidimensionnelles, et une valeur estimée du bruit qui correspond au deuxième cycle de traitement et qui correspond à chaque direction dimensionnelle, dans lequel un (quatrième) coefficient de filtre supplémentaire comprend une deuxième

valeur du coefficient de filtrage correspondant à chaque direction dimensionnelle ; et

le deuxième cycle de traitement est un cycle de traitement antérieur adjacent au premier cycle de traitement.

2. Procédé selon la revendication 1, dans lequel l'exécution (S120) d'un traitement de filtrage indépendant sur le signal de mouvement dans chaque direction dimensionnelle, et la détermination d'une valeur estimée de bruit correspondant à chaque direction dimensionnelle comprennent :

la détermination, en fonction d'un signal de mouvement dans une première direction dimensionnelle, d'un paramètre de mouvement correspondant à la première direction dimensionnelle, la première direction dimensionnelle étant une direction dimensionnelle quelconque des directions multidimensionnelles ; et

la détermination d'une valeur estimée du bruit correspondant à la première direction dimensionnelle en fonction du paramètre de mouvement et d'un premier coefficient de filtrage correspondant à la première direction dimensionnelle.

3. Procédé selon la revendication 2, dans lequel le premier coefficient de filtrage est un coefficient de filtrage qui correspond au premier cycle de traitement et qui correspond à la première direction dimensionnelle ; et

avant la détermination d'une valeur estimée de bruit correspondant à la première direction dimensionnelle en fonction du paramètre de mouvement et d'un premier coefficient de filtrage correspondant à la première direction dimensionnelle, le procédé comprend en outre :

l'obtention du premier coefficient de filtrage en fonction d'un signal d'onde d'impulsion cible qui correspond au deuxième cycle de traitement et qui comprend un bruit de mouvement, un paramètre de mouvement cible qui correspond au deuxième cycle de traitement et qui correspond à la première direction dimensionnelle, et un troisième coefficient de filtrage qui correspond au deuxième cycle de traitement et qui correspond à la première direction dimensionnelle.

4. Procédé selon la revendication 3, dans lequel l'obtention du premier coefficient de filtrage en fonction d'un signal d'onde d'impulsion cible qui correspond à un deuxième cycle de traitement et qui comprend un bruit de mouvement, un paramètre de mouvement cible qui correspond au deuxième cycle de traitement et qui correspond à la première direction dimensionnelle, et un troisième coefficient de filtrage qui correspond au deuxième cycle de traitement et qui correspond à la première direction dimensionnelle comprend :

l'obtention d'un signal d'erreur correspondant à la première direction dimensionnelle en fonction du signal d'onde d'impulsion cible, du paramètre de mouvement cible et du troisième coefficient de filtrage ;

l'obtention d'un coefficient d'ajustement du filtre en fonction du signal d'erreur et du paramètre de mouvement cible ; et

l'obtention du premier coefficient de filtrage en fonction du coefficient d'ajustement du filtre et du troisième coefficient de filtrage.

**5.** Appareil de traitement des signaux (300), **caractérisé en ce qu'**il comprend :

un module d'obtention (310), configuré pour obtenir un premier signal d'onde d'impulsion et des signaux de mouvement dans des directions multidimensionnelles, le premier signal d'onde d'impulsion comprenant le bruit de
mouvement,
dans lequel le premier signal d'onde d'impulsion est l'information d'onde d'impulsion acquise par un appareil d'acquisition de signaux du dispositif électronique,
les signaux de mouvement dans des directions
multidirectionnelles sont les signaux de mouvement dans les directions multidimensionnelles correspondantes du dispositif électronique, acquis par un capteur du dispositif électronique ;
un premier module de traitement
(320), configuré pour effectuer un traitement de filtrage indépendant sur le signal de mouvement dans chaque direction dimensionnelle, et déterminer une valeur estimée de bruit correspondant à chaque direction dimensionnelle ; et
un deuxième module de traitement (330), configuré pour utiliser les valeurs estimées du bruit dans les directions multidimensionnelles comme paramètres d'entrée parallèles, utiliser le premier signal d'onde d'impulsion comme paramètre d'entrée et effectuer un traitement de filtrage sur le premier signal d'onde d'impulsion, afin d'obtenir un deuxième signal d'onde d'impulsion ;
dans lequel le deuxième module de traitement (330) est configuré pour utiliser les valeurs estimées du bruit dans la direction multidimensionnelle comme paramètres d'entrée parallèles, pour utiliser le premier signal d'onde d'impulsion comme paramètre d'entrée et pour obtenir un (deuxième)
coefficient de filtrage correspondant aux
directions multidimensionnelles, dans lequel le (deuxième)
coefficient de filtrage comprend une première valeur
de coefficient de filtrage correspondant à chaque direction dimensionnelle, et la réalisation d'un traitement de filtrage sur le premier signal d'onde d'impulsion en fonction de la valeur estimée du bruit dans chaque direction dimensionnelle et de la première valeur du coefficient de filtrage correspondant à chaque direction dimensionnelle, afin d'obtenir le deuxième signal d'onde d'impulsion ;
dans lequel le (deuxième)
coefficient de filtrage est un coefficient de filtrage qui correspond à un premier cycle de traitement et qui correspond aux directions multidimensionnelles ; et
le deuxième module de traitement (330) est configuré pour obtenir le (deuxième)
coefficient de filtrage
en fonction d'un signal d'onde d'impulsion correspondant à un deuxième cycle de traitement et obtenu par traitement de filtrage, un autre (quatrième)
coefficient de filtrage qui correspond au deuxième
cycle de traitement et qui correspond aux directions multidimensionnelles, et une valeur estimée du bruit qui correspond au deuxième cycle de traitement et qui correspond à chaque direction dimensionnelle, dans lequel l'autre (quatrième)
coefficient de filtrage comprend une deuxième valeur de coefficient de filtrage correspondant à chaque direction dimensionnelle et le deuxième cycle de traitement est un cycle de traitement antérieur adjacent au premier cycle de traitement.

**6.** Appareil (300) selon la revendication 5, dans lequel le premier module de traitement (320) est configuré pour déterminer, en fonction d'un signal de mouvement dans une première direction dimensionnelle, un paramètre de mouvement correspondant à la première direction dimensionnelle, dans lequel la première direction dimensionnelle est une direction dimensionnelle quelconque des directions multidimensionnelles ; et déterminer une valeur estimée de bruit correspondant à la première direction dimensionnelle en fonction du paramètre de mouvement et d'un premier coefficient de filtrage correspondant à la première direction dimensionnelle.

**7.** Appareil (300) selon la revendication 6, dans lequel le premier coefficient de filtrage est un coefficient de filtrage qui correspond au premier cycle de traitement et qui correspond à la première direction dimensionnelle ; et le premier module de traitement (320) est configuré pour : avant la détermination d'une valeur estimée de bruit correspondant à la première direction dimensionnelle en fonction du paramètre de mouvement et d'un premier coefficient de filtrage correspondant à la première direction dimensionnelle, obtenir le premier coefficient de filtrage en fonction d'un signal d'onde d'impulsion cible correspondant au deuxième cycle de traitement et comprenant un bruit de mouvement, d'un paramètre de mouvement cible correspondant au deuxième cycle de traitement et correspondant à la première direction dimensionnelle, et d'un troisième coefficient de filtrage correspondant au deuxième cycle de traitement et correspondant à la première direction dimensionnelle.

8. Appareil (300) selon la revendication 7, dans lequel le premier module de traitement (320) est configuré pour obtenir un signal d'erreur correspondant à la première direction dimensionnelle en fonction du signal d'onde d'impulsion cible, du paramètre de mouvement cible et du troisième coefficient de filtrage ; obtenir un coefficient d'ajustement du filtre en fonction du signal d'erreur et du paramètre de mouvement cible et obtenir le premier coefficient de filtrage en fonction du coefficient d'ajustement du filtre et du troisième coefficient de filtrage.

9. Support de stockage lisible stockant un programme ou une instruction qui, lorsqu'ils sont exécutés par un ordinateur, amènent ledit ordinateur à effectuer les étapes du procédé de traitement des signaux selon l'une quelconque des revendications 1 à 4.

10. Produit de programme informatique comprenant des instructions qui, lorsque le programme est exécuté par un ordinateur, amènent l'ordinateur à exécuter le procédé de traitement des signaux selon l'une quelconque des revendications 1 à 4.

Obtain a first pulse wave signal and motion signals in multi-dimensional directions, where the first pulse wave signal includes motion noise — S110

Perform independent filtering processing on the motion signal in each dimensional direction, and determine a noise estimated value corresponding to each dimensional direction — S120

Use the noise estimated values in the multi-dimensional directions as parallel input parameters, use the first pulse wave signal as an input parameter, and perform filtering processing on the first pulse wave signal, to obtain a second pulse wave signal — S130

FIG. 1

PPG signal including motion artifact noise d(n)

Acceleration signal along an X axis

x(n)

NLMS adaptive filter

Acceleration signal along a Y axis

y(n)

NLMS adaptive filter

Acceleration signal along a Z axis

z(n)

NLMS adaptive filter

+
−

+
−

+
−

NLMS adaptive filter

Heart rate estimation signal

+
−

e(n)

FIG. 2

300

310 320

| Obtaining module | First processing module |

330

| Second processing module |

Signal processing apparatus

## FIG. 3

400

Electronic device

401 402

| Processor | ⟺ | Memory |

## FIG. 4

1000

Radio frequency
unit —— 1001

Network module —— 1002

1010

Memory —— 1009

Application

Operating
system

Processor

Audio output unit —— 1003

Input unit —— 1004

Graphics
processing
unit —— 10041

Microphone —— 10042

Interface unit —— 1008

User input unit —— 1007

Touch panel —— 10071

Another input
device —— 10072

1006

Display unit

Display
panel —— 10061

Sensor —— 1005

FIG. 5

**EP 4 353 146 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019008458 A1

- US 2009005695 A1

**Non-patent literature cited in the description**

- **CHOWDHURY SAYEED SHAFAYET et al.** *Real-Time Robust Heart Rate Estimation From Wrist-Type PPG Signals Using Multiple Reference Adaptive Noise Cancellation*